# EUROPEAN PATENT APPLICATION

(11) **EP 4 725 408 A1**
(43) Date of publication of application: **15.04.2026**
(21) Application number: 25207669.0
(22) Date of filing: 09.10.2025
(51) Int. Cl.: A61B 5/024, A61B 5/00

(54) **METHOD FOR PROCESSING SIGNAL, METHOD FOR ACQUIRING DATA, AND ELECTRONIC DEVICE AND STORAGE MEDIUM**

(30) Priority: 11.10.2024 CN 202411419675
(71) Applicant: Shenzhen Goodix Technology Co., Ltd., Shenzhen, Guangdong 518045 (CN)
(72) Inventor: WAN, Peng, Guangdong, 518045 (CN)
(74) Representative: Kraus & Lederer PartGmbB

(57) **Abstract**

A method for processing a signal, a method for acquiring data, and an electronic device are provided. The method includes: processing, based on first parameter information stored in a parameter register, an analog signal obtained by sampling a to-be-measured signal to obtain a first digital signal; replacing, when receiving second parameter information sent by a second processing module, the first parameter information in the parameter register with the second parameter information; and processing the analog signal obtained by sampling the to-be-measured signal, based on the second parameter information stored in the parameter register to obtain a second digital signal, and setting a target identifier for a first digital signal of the second digital signal obtained through processing based on the second parameter information.

## Description

### TECHNICAL FIELD

Embodiments of the present disclosure relate to the field of data processing, in particularly to a method for processing a signal, a method for acquiring data and an electronic device.

### BACKGROUND

Currently, in the process of signal collection for analog signals, considering factors such as quality of the collected signals and costs, it is common practice to convert analog signals into digital signals for collection.

For example, in the process of collecting a photoplethysmography (PPG) signal, a light-emitting component may emit light to illuminate the skin surface, and a receiving component may receive a reflected light signal and convert the light signal into a PPG signal. An analog front end may convert this PPG signal into a corresponding digital signal based on parameter information sent by a microcontroller unit. After acquiring the digital signal corresponding to the PPG signal, the microcontroller unit may restore the digital signal based on the current parameter information to obtain a measurement value of the PPG signal, thereby achieving the collection of the PPG signal. In addition, the microcontroller unit may also determine whether the current parameter information needs to be updated based on the measurement value of the PPG signal.

However, after the microcontroller unit sends updated parameter information to the analog front end, if the parameter information in the analog front end fails to be updated promptly, the analog front end may still determine the digital signal corresponding to the PPG signal based on the pre-update parameter information. Meanwhile, the microcontroller unit will restore the digital signal based on the updated parameter information, which may result in significant errors in the obtained measurement value of the PPG signal, thus causing the problem of significant signal processing errors.

### SUMMARY

In view of the above, the present disclosure provides a method for processing a signal, a method for acquiring data, a processing module, an electronic device, and a storage medium to at least partially address the aforementioned issues.

According to a first aspect of embodiments of the present disclosure, a method for processing a signal is provided, applied to a first processing module, the method including: processing, based on first parameter information stored in a parameter register, an analog signal obtained by sampling a to-be-measured signal to obtain a first digital signal; replacing, when receiving second parameter information sent by a second processing module, the first parameter information in the parameter register with the second parameter information; and processing the analog signal obtained by sampling the to-be-measured signal, based on the second parameter information stored in the parameter register to obtain a second digital signal, and setting a target identifier for the first digital signal of the second digital signal obtained through processing based on the second parameter information; where, the target identifier is used to indicate to the second processing module to process the first digital signal based on the first parameter information, to obtain a first target measurement value of the to-be-measured signal, and process the second digital signal and a digital signal following the second digital signal based on the second parameter information, to obtain a second target measurement value of the to-be-measured signal.

According to a second aspect of embodiments of the present disclosure, a method for acquiring data is provided, applied to a second processing module, the method for acquiring data including: acquiring at least one digital signal from a head of a queue stored in a first processing module, where, the digital signal is obtained by the first processing module by processing an analog signal obtained by sampling a to-be-measured signal, and digital signals in the queue are arranged in an order in which analog signals corresponding to the digital signals are sampled; determining whether the at least one digital signal includes a digital signal that is provided with a target identifier , wherein at most one digital signal provided with the target identifier is included in the at least one digital signal, the target identifier is used to indicate that among the at least one digital signal, each digital signal preceding the digital signal provided with the target identifier isa first digital signal, the digital signal provided with the target identifier and each digital signal following the digital signal provided with the target identifier are a second digital signal, the first digital signal is obtained through processing by the first processing module based on first parameter information, and the second digital signal is obtained through processing by the first processing module based on second parameter information; and processing, when the at least one digital signal includes the digital signal that is provided with the target identifier, the first digital signal among the at least one digital signal, based on the first parameter information, to obtain a first target measurement value of the to-be-measured signal, and processing the second digital signal among the at least one digital signal, based on the second parameter information, to obtain a second target measurement value of the to-be-measured signal.

According to a third aspect of embodiments of the present disclosure, a first processing module is provided, including: a first processing unit, configured to process, based on first parameter information stored in a parameter register, an analog signal obtained by sampling a to-be-measured signal to obtain a first digital signal; an information replacement unit, configured to replace, when receiving second parameter information sent by a second processing module, the first parameter information in the parameter register with the second parameter information; and a second processing unit, configured to process the analog signal obtained by sampling the to-be-measured signal, based on the second parameter information stored in the parameter register to obtain a second digital signal, and set a target identifier for the first digital signal of the second digital signal obtained through processing based on the second parameter information; where the target identifier is used to indicate to the second processing module to process the first digital signal based on the first parameter information, to obtain a first target measurement value of the to-be-measured signal, and process the second digital signal and a digital signal following the second digital signal based on the second parameter information, to obtain a second target measurement value of the to-be-measured signal.

According to a fourth aspect of embodiments of the present disclosure, a second processing module is provided, including: an information acquisition unit, configured to acquire at least one digital signal from a head of a queue stored in a first processing module, where, the digital signal is obtained by the first processing module by processing an analog signal obtained by sampling a to-be-measured signal, and digital signals in the queue are arranged in an order in which analog signals corresponding to the digital signals are sampled; a signal determination unit, configured to determine whether the at least one digital signal includes a digital signal that is provided with a target identifier, wherein at most one digital signal provided with the target identifier is included in the at least one digital signal, the target identifier is used to indicate that among the at least one digital signal, each digital signal preceding the digital signal provided with the target identifier is a first digital signal. The digital signal provided with the target identifier and each digital signal following the digital signal provided with the target identifier are a second digital signal. The first digital signal is obtained through processing by the first processing module based on first parameter information, and the second digital signal is obtained through processing by the first processing module based on second parameter information; and a data acquisition unit, configured to process, when the at least one digital signal includes a digital signal that is provided with a target identifier, the first digital signal among the at least one digital signal, based on the first parameter information, to obtain a first target measurement value of the to-be-measured signal, and process the second digital signal among the at least one digital signal, based on the second parameter information, to obtain a second target measurement value of the to-be-measured signal.

According to a fifth aspect of embodiments of the present disclosure, an electronic device is provided, including: a processor, a memory, a communications interface and a communications bus, where the processor, the memory and the communications interface communicate with each other via the communications bus; and the memory is used to store at least one executable instruction, and the executable instruction causes the processor to perform operations corresponding to the method in the first aspect or operations corresponding to the method in the second aspect.

According to a sixth aspect of embodiments of the present disclosure, a computer storage medium is provided, storing a computer program thereon, the program, when executed by a processor, performs the method in the first aspect or the method in the second aspect.

According to a seventh aspect of embodiments of the present disclosure, a computer program product is provided, including computer instructions, the computer instructions, instruct a computing device to perform the method in the first aspect or the method in the second aspect.

In the solution for processing a signal provided by embodiments of the present disclosure, the first processing module processes the analog signal obtained by sampling the to-be-measured signal based on the first parameter information stored in the parameter register, to obtain the first digital signal, then when receiving the second parameter information sent by the second processing module, replaces the first parameter information in the parameter register with the second parameter information, processes the analog signal obtained by sampling the to-be-measured signal, based on the second parameter information stored in the parameter register to obtain the second digital signal, and sets the target identifier for the first digital signal of the second digital signal obtained through processing based on the second parameter information. Thus, the target identifier may be used to distinguish between the first digital signal obtained through processing based on the first parameter information and the second digital signal obtained through processing based on the second parameter information, enabling the second processing module to process the first digital signal based on the first parameter information, and also to process the second digital signal based on the second parameter information, which reduces the likelihood of processing the second digital signal based on the first parameter information, thereby reducing signal processing errors.

### BRIEF DESCRIPTION OF DRAWINGS

In order to more clearly illustrate the technical solution in embodiments of the present disclosure or the prior art, the accompanying drawings to be used in the description of the embodiments or the prior art will be briefly introduced below. Obviously, the accompanying drawings in the following description are only some of the embodiments recorded in the embodiments of the present disclosure, and those of ordinary skill in the art may also obtain other accompanying drawings based on these accompanying drawings.
Fig. 1 is a flowchart of a method for processing a signal according to an embodiment of the present disclosure;
Fig. 2 is a schematic diagram of a first processing module and a second processing module according to an embodiment of the present disclosure;
Fig. 3 is a processing sequence diagram of the first processing module according to an embodiment of the present disclosure;
Fig. 4 is a processing sequence diagram of the first processing module according to another embodiment of the present disclosure;
Fig. 5 is a flowchart of a method for acquiring data according to an embodiment of the present disclosure;
Fig. 6 is a schematic diagram of a first processing module according to an embodiment of the present disclosure;
Fig. 7 is a schematic diagram of a second processing module according to an embodiment of the present disclosure; and
Fig. 8 is a schematic diagram of an electronic device according to an embodiment of the present disclosure.

### DETAILED DESCRIPTION

### Method for processing a signal

An embodiment of the present disclosure provides a method for processing a signal. The method for processing a signal is applied to a first processing module. The first processing module may be an analog front end (AFE), or the like, which is not limited in embodiments of the present disclosure.

The method for processing a signal will be described in detail through multiple embodiments as follows.

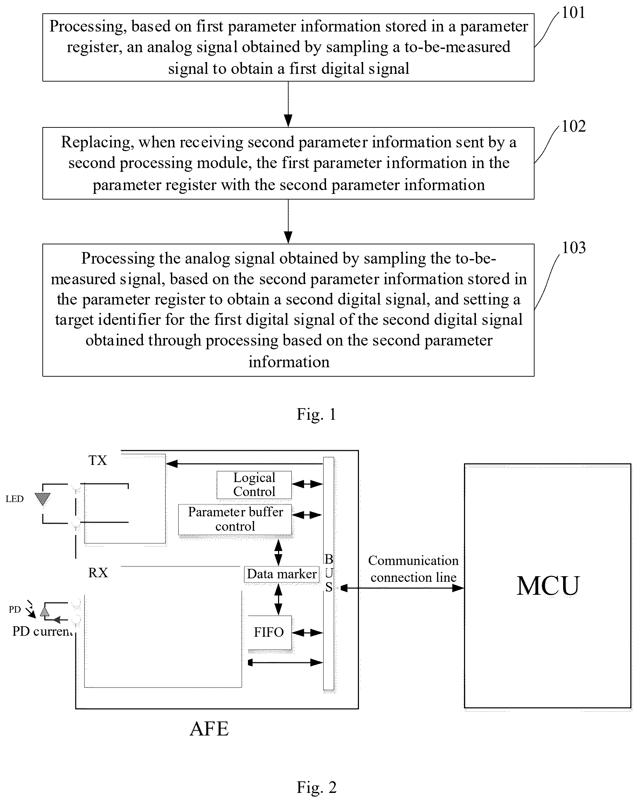

Fig. 1 is a flowchart of a method for processing a signal according to an embodiment of the present disclosure. As shown in Fig. 1, the method for processing a signal includes the following steps:

Step 101, processing, based on first parameter information stored in a parameter register, an analog signal obtained by sampling a to-be-measured signal to obtain a first digital signal.

Here, the to-be-measured signal is an analog signal, such as a continuous or discontinuous analog PPG signal. The parameter register is provided in the first processing module.

In a specific embodiment, the to-be-measured signal is periodically sampled based on the first processing module. For the analog signal obtained from the current sampling instance, processing may be performed based on the first parameter information currently stored in the parameter register to obtain the first digital signal.

Step 102, replacing, when receiving second parameter information sent by a second processing module, the first parameter information in the parameter register with the second parameter information.

Here, the second processing module may be a microcontroller unit (MCU), or the like.

In a specific embodiment, if the second processing module detects that the first parameter information needs to be updated, the second processing module sends the second parameter information to a buffer in the first processing module. After the second processing module sends the second parameter information, and before the next sampling of the to-be-measured signal, the first processing module may replace the first parameter information in the parameter register with the received second parameter information to realize the update of the parameter information.

Step 103, processing the analog signal obtained by sampling the to-be-measured signal, based on the second parameter information stored in the parameter register to obtain a second digital signal, and setting a target identifier for the first digital signal of the second digital signal obtained through processing based on the second parameter information.

Here, the target identifier is used to indicate to the second processing module to process the first digital signal based on the first parameter information, to obtain a first target measurement value of the to-be-measured signal, and process the second digital signal and a digital signal following the second digital signal based on the second parameter information, to obtain a second target measurement value of the to-be-measured signal.

It should be noted that the digital signal following the second digital signal refers to a digital signal obtained by the first processing module, by processing the analog signal obtained by sampling the to-be-measured signal, based on the second parameter information, which is obtained after the first digital signal of the second digital signal and before the second parameter information in the parameter register is updated.

In a specific embodiment, the to-be-measured signal is periodically sampled based on the first processing module, after the first parameter information in the parameter register is updated to the second parameter information, the first processing module may process the analog signal obtained by sampling the to-be-measured signal based on the second parameter information stored in the parameter register, to obtain the second digital signal, and set the target identifier for the first digital signal of the second digital signal obtained, enabling the second processing module to process the first digital signal based on the first parameter information, to obtain the first target measurement value of the to-be-measured signal corresponding to the first digital signal, and the second processing module can also process the second digital signal and the digital signal following the second digital signal based on the second parameter information, to obtain the second target measurement value of the corresponding to-be-measured signal.

In addition, a non-target identifier may be set for each digital signal other than the first digital signal of the second digital signal (whether the first digital signal or the second digital signal), and the non-target identifier is different from the target identifier. For example, the non-target identifier is 0, and the target identifier is 1.

In this embodiment of the present disclosure, the first processing module processes the analog signal obtained by sampling the to-be-measured signal based on the first parameter information stored in the parameter register, to obtain the first digital signal, then when receiving the second parameter information sent by the second processing module, replaces the first parameter information in the parameter register with the second parameter information, processes the analog signal obtained by sampling the to-be-measured signal, based on the second parameter information stored in the parameter register to obtain the second digital signal, and sets the target identifier for the first digital signal of the second digital signal obtained through processing based on the second parameter information. Thus, the target identifier may be used to distinguish between the first digital signal obtained through processing based on the first parameter information and the second digital signal obtained through processing based on the second parameter information, enabling the second processing module to process the first digital signal based on the first parameter information, and also to process the second digital signal based on the second parameter information, which reduces the likelihood of processing the second digital signal based on the first parameter information, thereby reducing signal processing errors.

In a possible implementation, the to-be-measured signal is a photoplethysmography (PPG) signal, the analog signal is a current signal, the first digital signal is a voltage signal, and the second digital signal is a voltage signal.

Fig. 2 is a schematic diagram of a first processing module and a second processing module according to an embodiment of the present disclosure. As shown in Fig. 2, the first processing module AFE includes a TX unit connected to a light-emitting diode (LED) and an RX unit connected to a photodiode (PD). The LED is used to emit light to illuminate the skin surface under the drive of a driver (DRV) included in the TX unit. The PD is used to receive a reflected and returned light signal and convert the light signal into a PD current signal. This PD current signal constitutes the PPG signal. The AFE periodically samples the PPG signal via the RX unit to obtain the analog signal input to the RX unit. The RX unit outputs a digital voltage signal based on the analog signal, which is either the first digital signal or the second digital signal. The first digital signal and the second digital signal are sequentially stored to a queue FIFO in an order in which corresponding analog signals are sampled (digital signals stored in the FIFO are typically binary values). The second processing module MCU connected to the AFE is used to read data stored in the FIFO. In addition, the AFE further includes a logical control, a parameter buffer control, a data marker, and a bus. The logical control is used to control the periodic sampling of the PPG signal by the AFE via the RX unit; the parameter buffer control is used to control the reception of the second parameter information via the buffer of the AFE; the data marker is used to set the target identifier and the non-target identifier for the digital signals stored in the FIFO; and the bus is used to connect the MCU via a communication connection line, thereby realizing connection between the AFE and the MCU.

In particular, a sampling period of the first processing module for the to-be-measured signal may typically be set between 20 milliseconds and 60 milliseconds. For example, the sampling period of the first processing module for the to-be-measured signal may be set to 40 milliseconds, which is not limited in embodiments of the present disclosure.

In this embodiment of the present disclosure, by using a photoplethysmography signal as the to-be-measured signal, errors in processing photoplethysmography signals may be reduced.

In a possible implementation, the above step 101 includes the following processing: controlling, based on a first current value included in the first parameter information, a current cancellation unit included in the first processing module to output a current whose current value is equal to the first current value, to cancel at least a portion of the analog signal obtained by sampling the to-be-measured signal, to obtain a first canceled signal; and processing the first canceled signal based on the first parameter information, to obtain the first digital signal.

In a specific embodiment, as shown in Fig. 2, the RX unit includes a current cancellation unit dc cancel. In the process of processing the analog signal to obtain the first digital signal based on the first parameter information (including the first current value), the dc cancel is used to provide a current whose current value is equal to the first current value and in the opposite direction to the PD current, to cancel at least a portion of the analog signal obtained by sampling the to-be-measured signal, to obtain the first canceled signal, and the TX unit then obtains the first digital signal based on the first canceled signal. It should be noted that the first current value is relatively small compared to the analog signal's value. Therefore, the current output by the dc cancel does not cause the analog signal to reverse direction relative to the first canceled signal.

In this embodiment of the present disclosure, the output current of the current cancellation unit may cancel at least a portion of the analog signal, reducing the likelihood of errors caused by excessive analog current saturating the first processing module, thereby enabling more accurate signal processing.

In a possible implementation, the above processing the first canceled signal based on the first parameter information, to obtain the first digital signal, includes the following processing: adjusting a gain of a transimpedance amplifier included in the first processing module to a first gain included in the first parameter information; inputting the first canceled signal to the transimpedance amplifier to obtain a first voltage signal output by the transimpedance amplifier; and inputting the first voltage signal to an analog-to-digital converter included in the first processing module to obtain the first digital signal output by the analog-to-digital converter.

In a specific embodiment, as shown in Fig. 2, the RX unit further includes the transimpedance amplifier (TIA) and the analog-to-digital converter (ADC). In the process of processing the analog signal to obtain the first digital signal based on the first parameter information (which also includes the first gain), when obtaining the first canceled signal, the first canceled signal is input to the TIA. The TIA outputs an analog first voltage signal, and the ADC then converts the analog first voltage signal into the first digital signal.

In this embodiment of the present disclosure, the first processing module may convert the first canceled signal (which is a current signal) into the first voltage signal by means of the transimpedance amplifier, and then convert the analog first voltage signal into the first digital signal by means of the analog-to-digital converter, thereby implementing the solution for converting the analog signal obtained by sampling the PPG signal into a digital signal.

Based on this, a signal value of the first digital signal is ADC code1=(I1_{PD}-I1_{d}, _{cancel})*tia1, where the unit of ADC code1 LSB as the unit, where I1_{PD} is the signal value corresponding to the analog signal processed by the first processing module based on the first parameter information stored in the parameter register (with the unit being µA), I1_{dc cancel} is the first current value (with the unit being µA), and tia1 is the first gain (with the unit being LSB/µA).

In a possible implementation, the processing the analog signal obtained by sampling the to-be-measured signal, based on the second parameter information stored in the parameter register to obtain a second digital signal in the above step 103, includes the following processing: controlling, based on a second current value included in the second parameter information, a current cancellation unit included in the first processing module to output a current whose current value is equal to the second current value, to cancel at least a portion of the analog signal obtained by sampling the to-be-measured signal, to obtain a second canceled signal; and processing the second canceled signal based on the second parameter information, to obtain the second digital signal.

In a specific embodiment, as shown in Fig. 2, the RX unit includes a current cancellation unit dc cancel. In the process of processing the analog signal to obtain the second digital signal based on the second parameter information (including the second current value), the dc cancel is used to provide a current whose current value is equal to the second current value and in the opposite direction to the PD current, to cancel at least a portion of the analog signal obtained by sampling the to-be-measured signal, to obtain the second canceled signal, and the TX unit then obtains the second digital signal based on the second canceled signal. It should be noted that the second current value is relatively small compared to the analog signal's value. Therefore, the current output by the dc cancel does not cause the analog signal to reverse direction relative to the second canceled signal.

In this embodiment of the present disclosure, the output current of the current cancellation unit may cancel at least a portion of the analog signal, reducing the likelihood of errors caused by excessive analog current saturating the first processing module, thereby enabling more accurate signal processing.

In a possible implementation, the processing the second canceled signal based on the second parameter information, to obtain the second digital signal, includes the following processing: adjusting a gain of a transimpedance amplifier included in the first processing module to a second gain included in the second parameter information; inputting the second canceled signal to the transimpedance amplifier to obtain a second voltage signal output by the transimpedance amplifier; and inputting the second voltage signal to an analog-to-digital converter included in the first processing module to obtain the second digital signal output by the analog-to-digital converter.

In a specific embodiment, as shown in Fig. 2, the RX unit further includes the transimpedance amplifier (TIA) and the analog-to-digital converter (ADC). In the process of processing the analog signal to obtain the second digital signal based on the second parameter information (which also includes the second gain), when obtaining the second canceled signal, the second canceled signal is input to the TIA, so that the TIA outputs an analog second voltage signal, and the ADC then converts the analog second voltage signal into the second digital signal.

In this embodiment of the present disclosure, the first processing module may convert the second canceled signal (which is a current signal) into the second voltage signal by means of the transimpedance amplifier, and then convert the analog second voltage signal into the second digital signal by means of the analog-to-digital converter, thereby implementing the solution for converting the analog signal obtained by sampling the PPG signal into a digital signal.

Based on this, a signal value of the second digital signal is ADC code2=(I2_{PD}-I2_{dc cancel})*tia2, where the unit of ADC code2 is LSB, where I2_{PD} is the signal value of the analog signal processed by the first processing module based on the second parameter information stored in the parameter register (with the unit being µA), I2_{dc cancel} is the second current value (with the unit being µA), and tia2 is the second gain (with the unit being LSB/µA).

The first processing module may adjust the gain of the transimpedance amplifier and the output current of the current cancellation unit by updating the parameter information stored in the parameter register. Then, a measurement range of the first processing module may be adjusted, allowing the first processing module to obtain more valid data during processing the PPG signal.

In a possible implementation, the method for processing a signal further includes: if, starting from receipt of a start command sent by the second processing module, an end command sent by the second processing module is received within waiting time, determining that the second parameter information sent by the second processing module has been received, where, the start command is used to indicate start of sending the second parameter information, and the end command is used to indicate end of sending the second parameter information; and if, starting from the receipt of the start command, the end command is not received within the waiting time, determining that the second parameter information sent by the second processing module has been received, when the waiting time has elapsed since the receipt of the start command.

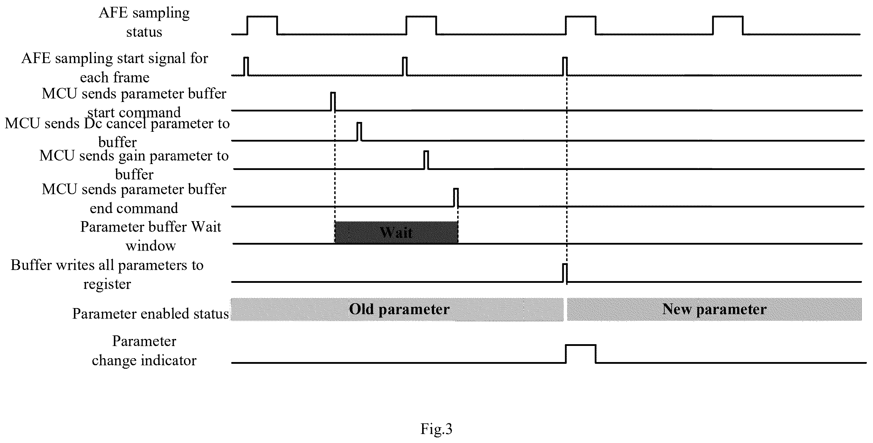

Fig. 3 is a processing sequence diagram of the first processing module according to an embodiment of the present disclosure. As shown in Fig. 3, old parameter refers to the first parameter information, and new parameter refers to the second parameter information. A bar length labeled with "Wait" is used to indicate waiting time. Based on this, after the AFE performs a certain sampling on the PPG signal, the MCU sends a start command corresponding to the second parameter information to the AFE. Then, the MCU sends the second parameter information to the AFE within the waiting time, and sends an end command corresponding to the second parameter information to the AFE within the waiting time. In this regard, it may be determined that the AFE has received the second parameter information sent by the second processing module.

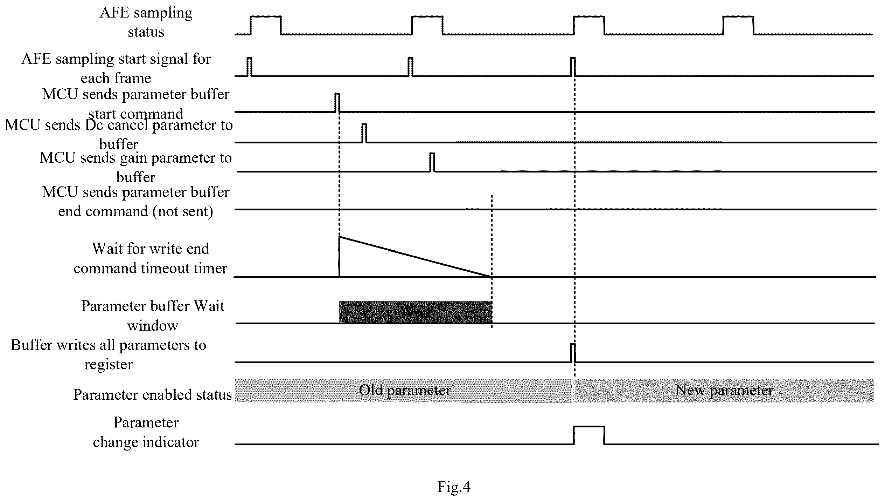

Fig. 4 is a processing sequence diagram of the first processing module according to another embodiment of the present disclosure. As shown in Fig. 4, old parameter refers to the first parameter information, and new parameter refers to the second parameter information. A bar length labeled with "Wait" is used to indicate waiting time. Based on this, after the AFE performs a certain sampling on the PPG signal, the MCU sends a start command corresponding to the second parameter information to the AFE. Then, the MCU sends the second parameter information to the AFE within the waiting time, but does not send an end command corresponding to the second parameter information to the AFE by the end of the waiting time. In this regard, it may also be determined that the AFE has received the second parameter information sent by the second processing module.

In this embodiment of the present disclosure, by adopting the solution where, if the end command is not received within the waiting time starting from the receipt of the start command, then determining that the second parameter information sent by the second processing module has been received when the waiting time has elapsed, the problem that the first parameter information cannot be updated due to the AFE failing to receive the end command caused by software or hardware issues may be avoided as much as possible.

### Method for acquiring data

An embodiment of the present disclosure provides a method for acquiring data. The method for acquiring data is applied to a second processing module. The second processing module may be a microcontroller unit (MCU), or the like, which is not limited in embodiments of the present disclosure.

The method for acquiring data will be described in detail through multiple embodiments as follows.

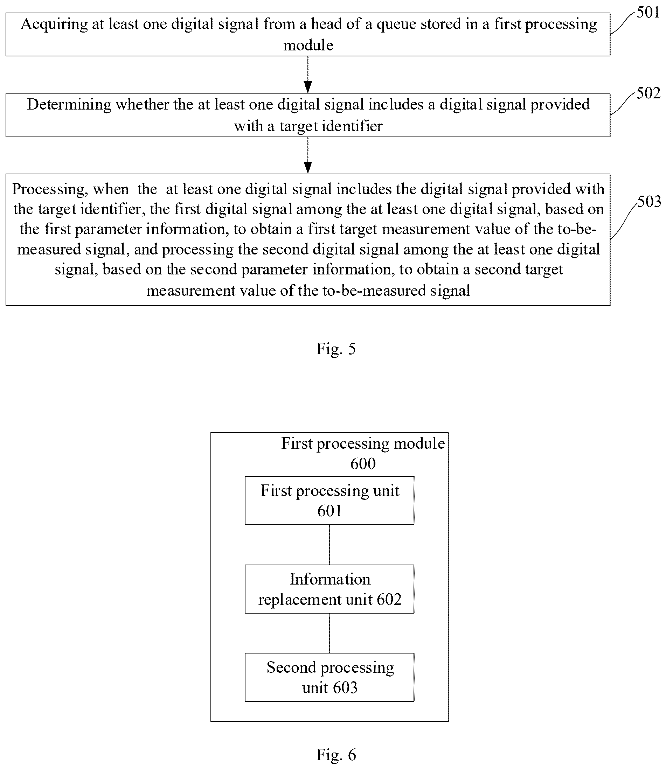

Fig. 5 is a flowchart of a method for acquiring data according to an embodiment of the present disclosure. As shown in Fig. 5, the method for acquiring data includes the following steps:

Step 501, acquiring at least one digital signal from a head of a queue stored in a first processing module.

Here, the digital signal is obtained by the first processing module by processing an analog signal obtained by sampling a to-be-measured signal, and digital signals in the queue are arranged in an order in which analog signals corresponding to the digital signals are sampled. The digital signal refers to either the first digital signal or the second digital signal in the aforementioned embodiments of the method for processing a signal.

It should be noted that step 501 in this embodiment of the present disclosure refers to a process of acquiring at least one digital signal from the head of the queue, which starts after first parameter information in a parameter register is updated to second parameter information and when the at least one digital signal acquired from the head of the queue includes at least a portion of the second digital signal, and ends before the at least one digital signal acquired from the head of the queue includes a third digital signal (the third digital signal is a digital signal obtained by the first processing module by processing the analog signal obtained by sampling the to-be-measured signal based on third parameter information stored in the parameter register used for replacing the second parameter information). Therefore, if the at least one digital signal is a single digital signal, then the digital signal is the second digital signal. If the at least one digital signal is multiple digital signals, then the multiple digital signals include at least one first digital signal and at least one second digital signal; or, all digital signals in the multiple digital signals are the second digital signal.

Step 502, determining whether the at least one digital signal includes a digital signal that is provided with a target identifier.

Here, at most one digital signal provided with the target identifier is included in the at least one digital signal, the target identifier is used to indicate that among the at least one digital signal, each digital signal preceding the digital signal provided with the target identifier is a first digital signal. The digital signal provided with the target identifier and each digital signal following the digital signal provided with the target identifier are a second digital signal. The first digital signal is obtained through processing by the first processing module based on first parameter information, and the second digital signal is obtained through processing by the first processing module based on second parameter information.

Step 503, processing, when the at least one digital signal includes the digital signal that is provided with the target identifier the first digital signal among the at least one digital signal, based on the first parameter information, to obtain a first target measurement value of the to-be-measured signal, and processing the second digital signal among the at least one digital signal, based on the second parameter information, to obtain a second target measurement value of the to-be-measured signal.

In this embodiment of the present disclosure, the at least one digital signal is acquired from the head of the queue stored in the first processing module, then it is determined whether the digital signal provided with the target identifier is included in the at least one digital signal, when the at least one digital signal includes the digital signal that is provided with the target identifier , the first digital signal among the at least one digital signal is processed based on the first parameter information, to obtain the first target measurement value of the to-be-measured signal, and the second digital signal among the at least one digital signal is processed based on the second parameter information, to obtain the second target measurement value of the to-be-measured signal. Thus, the target identifier may be used to distinguish between the first digital signal obtained through processing based on the first parameter information and the second digital signal obtained through processing based on the second parameter information by the first processing module, enabling the second processing module to process the first digital signal based on the first parameter information, and also to process the second digital signal based on the second parameter information, which reduces the likelihood of processing the second digital signal based on the first parameter information, thereby reducing signal processing errors, further reducing data acquisition errors.

In a possible implementation, the method for acquiring data further includes the following processing: when the at least one digital signal includes the digital signal that is provided with the target identifier, processing each digital signal among the at least one digital signal based on the second parameter information, to obtain a second target measurement value of the to-be-measured signal.

In a possible implementation, the to-be-measured signal is a photoplethysmography (PPG) signal, the analog signal is a current signal, and the digital signal is a voltage signal. As shown in Fig. 2, the signals have already been described above based on Fig. 2, detailed descriptions thereof will be omitted herein in this embodiment of the present disclosure.

In a possible implementation, the first digital signal is obtained by the first processing module by processing the analog signal obtained by sampling the to-be-measured signal, based on a first current value and a first gain included in the first parameter information, the first current value is used to indicate a current value of a current output by a current cancellation unit included in the first processing module, and the first gain is used to indicate a gain of a transimpedance amplifier included in the first processing module. Based on this, the processing the first digital signal among the at least one digital signal, based on the first parameter information, to obtain a first target measurement value of the to-be-measured signal in the above step 503, includes the following processing: processing the first digital signal among the at least one digital signal, based on the first current value and the first gain, to obtain the first target measurement value of the to-be-measured signal.

In a specific embodiment, the process of obtaining the first digital signal has been described above and detailed description thereof will be omitted in this embodiment of the present disclosure. Based on this, the first target measurement value I1 of the to-be-measured signal corresponding to the first digital signal is calculated as I1=ADC code1/tia1+I1_{dc cancel}. It follows that, under ideal conditions, the first target measurement value of the to-be-measured signal corresponding to the first digital signal equals I1_{PD}.

Thus, the calculation of the first target measurement value of the to-be-measured signal is relatively simple, which saves computing resources.

In a possible implementation, the second digital signal is obtained by the first processing module by processing the analog signal obtained by sampling the to-be-measured signal, based on a second current value and a second gain included in the second parameter information, the second current value is used to indicate a current value of a current output by a current cancellation unit included in the first processing module, and the second gain is used to indicate a gain of a transimpedance amplifier included in the first processing module. Based on this, the processing the second digital signal among the at least one digital signal, based on the second parameter information, to obtain a second target measurement value of the to-be-measured signal in the above step 503, includes the following processing: processing the second digital signal among the at least one digital signal, based on the second current value and the second gain, to obtain the second target measurement value of the to-be-measured signal.

In a specific embodiment, the process of obtaining the second digital signal has been described above and detailed description thereof will be omitted in this embodiment of the present disclosure. Based on this, the second target measurement value I2 of the to-be-measured signal corresponding to the second digital signal is calculated as I2=ADC code2/tia2+I2_{dc cancel}. It follows that, under ideal conditions, the second target measurement value of the to-be-measured signal corresponding to the second digital signal equals I2_{PD}.

Thus, the calculation of the second target measurement value of the to-be-measured signal is relatively simple, which saves computing resources.

Alternatively, after obtaining the second target measurement value of the to-be-measured signal, the second processing module may determine whether to update the second parameter information in the parameter register based on the second target measurement value. If yes, the second processing module may send parameter information for replacing the second parameter information to the first processing module, enabling the first processing module to adjust the gain of the transimpedance amplifier and the output current of the current cancellation unit.

### First processing module

Corresponding to the above embodiments of the method for processing a signal, Fig. 6 illustrates a schematic diagram of a first processing module according to an embodiment of the present disclosure. As shown in Fig. 6, the first processing module 600 includes:
a first processing unit 601, configured to process, based on first parameter information stored in a parameter register, an analog signal obtained by sampling a to-be-measured signal to obtain a first digital signal;
an information replacement unit 602, configured to replace, after receiving second parameter information sent by a second processing module, the first parameter information in the parameter register with the second parameter information; and
a second processing unit 603, configured to process the analog signal obtained by sampling the to-be-measured signal, based on the second parameter information stored in the parameter register to obtain a second digital signal, and set a target identifier for the first digital signal of the second digital signal obtained through processing based on the second parameter information;
where, the target identifier is used to indicate to the second processing module to process the first digital signal based on the first parameter information, to obtain a first target measurement value of the to-be-measured signal, and process the second digital signal and a digital signal following the second digital signal based on the second parameter information, to obtain a second target measurement value of the to-be-measured signal.

In this embodiment of the present disclosure, the first processing module 601 processes the analog signal obtained by sampling the to-be-measured signal based on the first parameter information stored in the parameter register, to obtain the first digital signal, then when receiving the second parameter information sent by the second processing module, the information replacement unit 602 replaces the first parameter information in the parameter register with the second parameter information, the second processing unit 603 processes the analog signal obtained by sampling the to-be-measured signal, based on the second parameter information stored in the parameter register to obtain the second digital signal, and sets the target identifier for the first digital signal of the second digital signal obtained through processing based on the second parameter information. Thus, the target identifier may be used to distinguish between the first digital signal obtained through processing based on the first parameter information and the second digital signal obtained through processing based on the second parameter information, enabling the second processing module to process the first digital signal based on the first parameter information, and also to process the second digital signal based on the second parameter information, which reduces the likelihood of processing the second digital signal based on the first parameter information, thereby reducing signal processing errors.

It should be noted that the first processing module in this embodiment is used to implement the corresponding method for processing a signal described in the aforementioned method embodiments and possesses the beneficial effects of the corresponding method embodiments, detailed description thereof will be omitted.

### Second processing module

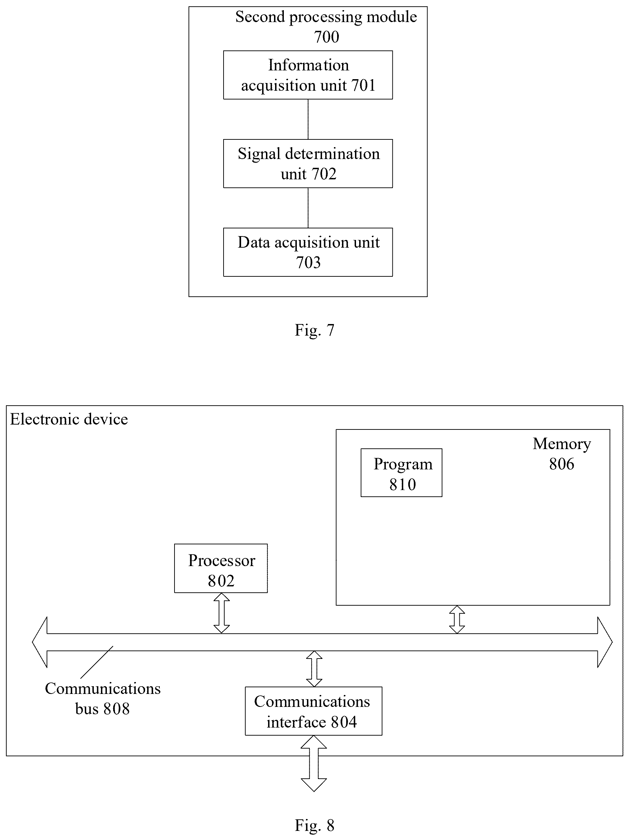

Corresponding to the above embodiments of the method for acquiring data, Fig. 7 illustrates a schematic diagram of a second processing module according to an embodiment of the present disclosure. As shown in Fig. 7, the second processing module 700 includes:
an information acquisition unit 701, configured to acquire at least one digital signal from a head of a queue stored in a first processing module, where, the digital signal is obtained by the first processing module by processing an analog signal obtained by sampling a to-be-measured signal, and digital signals in the queue are arranged in an order in which analog signals corresponding to the digital signals are sampled;
a signal determination unit 702, configured to determine whether the at least one digital signal includes a digital signal that is provided with a target identifier, wherein at most one digital signal provided with the target identifier is included in the at least one digital signal, the target identifier is used to indicate that among the at least one digital signal, each digital signal preceding the digital signal provided with the target identifier is a first digital signal. The digital signal provided with the target identifier and each digital signal following the digital signal provided with the target identifier area second digital signal. The first digital signal is obtained through processing by the first processing module based on first parameter information, and the second digital signal is obtained through processing by the first processing module based on second parameter information; and
a data acquisition unit 703, configured to process, when the at least one digital signal includes the digital signal that is provided with the target identifier , the first digital signal among the at least one digital signal, based on the first parameter information, to obtain a first target measurement value of the to-be-measured signal, and process the second digital signal among the at least one digital signal, based on the second parameter information, to obtain a second target measurement value of the to-be-measured signal.

In this embodiment of the present disclosure, the information acquisition unit 701 acquires the at least one digital signal from the head of the queue stored in the first processing module, the signal determination unit 702 determines whether the at least one digital signal includes a digital signal that is provided with a target identifier. When the at least one digital signal includes the digital signal that is provided with the target identifier, the data acquisition unit 703 processes the first digital signal among the at least one digital signal based on the first parameter information, to obtain the first target measurement value of the to-be-measured signal, and processes the second digital signal among the at least one digital signal based on the second parameter information, to obtain the second target measurement value of the to-be-measured signal. Thus, the target identifier may be used to distinguish between the first digital signal obtained through processing based on the first parameter information and the second digital signal obtained through processing based on the second parameter information by the first processing module, enabling the second processing module to process the first digital signal based on the first parameter information, and also to process the second digital signal based on the second parameter information, which reduces the likelihood of processing the second digital signal based on the first parameter information, thereby reducing signal processing errors, further reducing data acquisition errors.

It should be noted that the second processing module in this embodiment is used to implement the corresponding method for acquiring data described in the aforementioned method embodiments and possesses the beneficial effects of the corresponding method embodiments, detailed description thereof will be omitted.

### Electronic device

Fig. 8 is a schematic block diagram of an electronic device according to an embodiment of the present disclosure. The specific embodiments of the present disclosure do not limit the specific implementation of the electronic device. As shown in Fig. 8, the electronic device may include: a processor 802, a communications interface 804, a memory 806, and a communications bus 808.

The processor 802, the communications interface 804, and the memory 806 communicate with each other via the communications bus 808.

The communications interface 804 is used to communication with other electronic devices or servers.

The processor 802 is used to execute a program 810, in particular may execute relevant steps in any one of the aforementioned embodiments of the method for processing a signal or relevant steps in any one of the aforementioned embodiments of the method for acquiring data.

In particular, the program 810 may include program code, which includes computer operation instructions.

The processor 802 may be a CPU, or an application specific integrated circuit (ASIC), or one or more integrated circuits configured to implement the embodiments of the present disclosure. Intelligent devices include one or more processors, which may be of the same type, such as one or more CPUs; or may be of different types, such as one or more CPUs and one or more ASICs.

RISC-V is an open-source instruction set architecture based on the principles of reduced instruction set computing (RISC), which may be applied across various domains, including microcontrollers and FPGA chips, and in particular in fields such as IoT security, industrial control, mobile phones, and personal computers. Due to its design considerations for compact size, high speed, and low power consumption, RISC-V is particularly well-suited for modern computing devices such as warehouse-scale cloud computers, high-end mobile phones, and micro-embedded systems. With the rise of Artificial Intelligence of Things (AIoT), the RISC-V instruction set architecture has also received increasing attention and support, and is expected to become a widely used CPU architecture in the next generation.

The computer operation instructions in the embodiments of the present disclosure may be computer operation instructions based on the RISC-V instruction set architecture. Correspondingly, the processor 802 may be designed based on the RISC-V instruction set. In particular, a chip of the processor in the electronic device provided by the embodiments of the present disclosure may be a chip designed using the RISC-V instruction set. The chip may execute executable code based on the configured instructions, thereby implementing the method for processing a signal or the method for acquiring data described in the above embodiments.

The memory 806 is used to store the program 810. The memory 806 may contain high-speed RAM memory, or may further include non-volatile memory, such as at least one disk memory.

The program 810 may be used to cause the processor 802 to perform the method for processing a signal or the method for acquiring data in any one of the aforementioned embodiments.

For the specific implementation of each step in the program 810, reference may be made to the corresponding descriptions in the relevant steps and units of any one of the aforementioned embodiments of the method for processing a signal or the method for acquiring data, detailed descriptions thereof will be omitted. Those skilled in the art may clearly understand that, for the convenience and conciseness of description, the specific working processes of the devices and modules described above may be referred to the corresponding process descriptions in the aforementioned method embodiments, detailed descriptions thereof will be omitted.

By using the electronic device according to an embodiment of the present disclosure, the target identifier may be used to distinguish between the first digital signal obtained through processing based on the first parameter information and the second digital signal obtained through processing based on the second parameter information, enabling the second processing module to process the first digital signal based on the first parameter information, and also to process the second digital signal based on the second parameter information, which reduces the likelihood of processing the second digital signal based on the first parameter information, thereby reducing signal processing errors.

### Computer storage medium

The present disclosure also provides a computer readable storage medium, storing instructions for causing a machine to perform the method for processing a signal or the method for acquiring data described herein. In particular, a system or apparatus equipped with the storage medium may be provided, on which software program code is stored for implementing functions of any one of the embodiments described above, enabling a computer (or CPU or MPU) of the system or apparatus to read and execute the program code stored on the storage medium.

In this case, the program code read from the storage medium itself may implement the functions of any one of the embodiments described above. Therefore, the program code and the storage medium storing the program code constitute part of the present disclosure.

The storage medium for providing the program code includes floppy disks, hard disks, magneto-optical disks, optical disks (such as CD-ROM, CD-R, CD-RW, DVD-ROM, DVD-RAM, DVD-RW, DVD+RW), magnetic tapes, non-volatile memory cards, and ROM. Alternatively, the program code may be downloaded from a server computer via a communication network.

### Computer program product

An embodiment of the present disclosure further provides a computer program product including computer instructions, the computer instructions, instruct a computing device to perform operations corresponding to any one of the above multiple method embodiments.

It should be noted that user-related information (including but not limited to user device information, user personal information, etc.) and data (including but not limited to sample data for model training, analytical data, stored data, displayed data, etc.) involved in the embodiments of the present disclosure are all authorized by the user or obtained through full authorization from all relevant parties. Furthermore, the collection, use, and processing of relevant data must comply with applicable laws, regulations, and standards of the relevant countries and regions. Corresponding operational interfaces shall be provided to enable users to choose to authorize or decline.

It should be noted that, based on implementation requirements, the various components/steps described in the embodiments of the present disclosure may be subdivided into additional components/steps. Alternatively, two or more components/steps, or portions of components/steps, may be combined to form new components/steps to achieve the objectives of the embodiments of the present disclosure.

The method according to the embodiments of the present disclosure may be implemented in hardware or firmware, or may be implemented as software or computer code stored on a recording medium (such as CD-ROM, RAM, floppy disk, hard disk, or magneto-optical disk), or as computer code originally stored on a remote recording medium or non-transitory machine-readable medium and downloaded via a network for storage on a local recording medium. Thus, the methods described herein may be stored as such software processing on a recording medium using a general-purpose computer, a dedicated processor, or programmable or dedicated hardware (such as an ASIC or FPGA). It may be understood that a computer, processor, microprocessor controller, or programmable hardware includes a storage component (e.g., RAM, ROM, flash memory, etc.) capable of storing or receiving software or computer code, and when the software or computer code is accessed and executed by the computer, processor, or hardware, the methods described herein are implemented. Furthermore, when a general-purpose computer accesses code for implementing the methods shown herein, execution of the code transforms the general-purpose computer into a specialized computer for performing the methods shown herein.

Those skilled in the art may recognize that the units and method steps of each example described in conjunction with the embodiments disclosed herein can be implemented using electronic hardware, or a combination of computer software and electronic hardware. Whether these functions are executed in hardware or software depends on the specific application and design constraints of the technical solution. Those skilled in the art may use different methods to implement the described functions for specific applications, however, such implementations should not be considered outside the scope of the embodiments of the present disclosure.

The above embodiments are provided merely to illustrate the embodiments of the present disclosure and are not intended to limit the scope of the embodiments of the present disclosure. Those skilled in the relevant technical field may make various changes and modifications without departing from the spirit and scope of the embodiments of the present disclosure. Therefore, all equivalent technical solutions also fall within the scope of the embodiments of the present disclosure. The scope of patent protection for the embodiments of the present disclosure shall be defined by the appended claims.

## Claims

1. A method for processing a signal, applied to a first processing module, the method comprising:
processing, based on first parameter information stored in a parameter register, an analog signal obtained by sampling a to-be-measured signal to obtain a first digital signal;
replacing, when receiving second parameter information sent by a second processing module, the first parameter information in the parameter register with the second parameter information; and
processing the analog signal obtained by sampling the to-be-measured signal, based on the second parameter information stored in the parameter register to obtain a second digital signal, and setting a target identifier for a first digital signal of the second digital signal obtained through processing based on the second parameter information;
wherein, the target identifier is used to indicate to indicate to the second processing module to process the first digital signal based on the first parameter information, to obtain a first target measurement value of the to-be-measured signal, and process the second digital signal based on the second parameter information, to obtain a second target measurement value of the to-be-measured signal.

2. The method according to claim 1, wherein, the to-be-measured signal is a photoplethysmography (PPG) signal, the analog signal is a current signal, the first digital signal is a voltage signal, and the second digital signal is a voltage signal.

3. The method according to claim 2, wherein, the processing, based on first parameter information stored in a parameter register, an analog signal obtained by sampling a to-be-measured signal to obtain a first digital signal, comprises:
controlling, based on a first current value comprised in the first parameter information, a current cancellation unit comprised in the first processing module to output a current whose current value is equal to the first current value, to cancel at least a portion of the analog signal obtained by sampling the to-be-measured signal, to obtain a first canceled signal; and
processing the first canceled signal based on the first parameter information, to obtain the first digital signal.

4. The method according to claim 3, wherein, the processing the first canceled signal based on the first parameter information, to obtain the first digital signal, comprises:
adjusting a gain of a transimpedance amplifier comprised in the first processing module to a first gain comprised in the first parameter information;
inputting the first canceled signal to the transimpedance amplifier to obtain a first voltage signal output by the transimpedance amplifier; and
inputting the first voltage signal to an analog-to-digital converter comprised in the first processing module to obtain the first digital signal output by the analog-to-digital converter.

5. The method according to claim 2, wherein, the processing the analog signal obtained by sampling the to-be-measured signal, based on the second parameter information stored in the parameter register to obtain a second digital signal, comprises:
controlling, based on a second current value comprised in the second parameter information, a current cancellation unit comprised in the first processing module to output a current whose current value is equal to the second current value, to cancel at least a portion of the analog signal obtained by sampling the to-be-measured signal, to obtain a second canceled signal; and
processing the second canceled signal based on the second parameter information, to obtain the second digital signal.

6. The method according to claim 5, wherein, the processing the second canceled signal based on the second parameter information, to obtain the second digital signal, comprises:
adjusting a gain of a transimpedance amplifier comprised in the first processing module to a second gain comprised in the second parameter information;
inputting the second canceled signal to the transimpedance amplifier to obtain a second voltage signal output by the transimpedance amplifier; and
inputting the second voltage signal to an analog-to-digital converter comprised in the first processing module to obtain the second digital signal output by the analog-to-digital converter.

7. The method according to claim 1, wherein the method further comprises:
if, starting from receipt of a start command sent by the second processing module, an end command sent by the second processing module is received within waiting time, determining that the second parameter information sent by the second processing module has been received, wherein, the start command is used to indicate start of sending the second parameter information, and the end command is used to indicate end of sending the second parameter information; and
if, starting from the receipt of the start command, the end command is not received within the waiting time, determining that the second parameter information sent by the second processing module has been received, when the waiting time has elapsed since the receipt of the start command.

8. A method for acquiring data, applied to a second processing module, the method for acquiring data comprising:
acquiring at least one digital signal from a head of a queue stored in a first processing module, wherein the at least one digital signal is obtained by the first processing module by processing an analog signal that is obtained by sampling a to-be-measured signal, and the at least one digital signal in the queue is arranged in an order in which the analog signals corresponding to the digital signal is sampled;
determining whether the at least one digital signal includes a digital signal that is provided with a target identifier, wherein at most one digital signal provided with the target identifier is included in the at least one digital signal, the target identifier is used to indicate that among the at least one digital signal, each digital signal preceding the digital signal provided with the target identifier is a first digital signal, the digital signal provided with the target identifier and each digital signal following the digital signal provided with the target identifier area second digital signal, the first digital signal is obtained through processing by the first processing module based on first parameter information, and the second digital signal is obtained through processing by the first processing module based on second parameter information; and
processing, when the at least one digital signal includes the digital signal that is provided with the target identifier, the first digital signal among the at least one digital signal, based on the first parameter information, to obtain a first target measurement value of the to-be-measured signal, and processing the second digital signal among the at least one digital signal, based on the second parameter information, to obtain a second target measurement value of the to-be-measured signal.

9. The method according to claim 8, wherein the method further comprises:
processing, when the at least one digital signal does not include the digital signal provided with the target identifier, each digital signal among the at least one digital signal based on the second parameter information, to obtain a second target measurement value of the to-be-measured signal.

10. The method according to claim 8 or 9, wherein the to-be-measured signal is a photoplethysmography (PPG) signal, the analog signal is a current signal, and the digital signal is a voltage signal.

11. The method according to claim 10, wherein the first digital signal is obtained by the first processing module by processing the analog signal obtained by sampling the to-be-measured signal, based on a first current value and a first gain comprised in the first parameter information, the first current value is used to indicate a current value of a current output by a current cancellation unit comprised in the first processing module, and the first gain is used to indicate a gain of a transimpedance amplifier comprised in the first processing module;
the processing the first digital signal among the at least one digital signal, based on the first parameter information, to obtain a first target measurement value of the to-be-measured signal, comprises:
processing the first digital signal among the at least one digital signal, based on the first current value and the first gain, to obtain the first target measurement value of the to-be-measured signal.

12. The method according to claim 10, wherein the second digital signal is obtained by the first processing module by processing the analog signal obtained by sampling the to-be-measured signal, based on a second current value and a second gain comprised in the second parameter information, the second current value is used to indicate a current value of a current output by a current cancellation unit comprised in the first processing module, and the second gain is used to indicate a gain of a transimpedance amplifier comprised in the first processing module;
the processing the second digital signal among the at least one digital signal, based on the second parameter information, to obtain a second target measurement value of the to-be-measured signal, comprises:
processing the second digital signal among the at least one digital signal, based on the second current value and the second gain, to obtain the second target measurement value of the to-be-measured signal.

13. An electronic device, comprising: a processor, a memory, a communications interface and a communications bus, wherein the processor, the memory and the communications interface communicate with each other via the communications bus; and
the memory is used to store at least one executable instruction, and the executable instruction causes the processor to perform operations corresponding to the method for processing a signal according to any one of claims 1-7 or the method for acquiring data according to any one of claims 8-12.

14. A computer storage medium, storing a computer program thereon, the program, when executed by a processor, implements the method for processing a signal according to any one of claims 1-7 or the method for acquiring data according to any one of claims 8-12.
